(19) Europäisches Patentamt
European Patent Office
Office européen des brevets

(11) Veröffentlichungsnummer: **0 207 415**
**B1**

(12) # EUROPÄISCHE PATENTSCHRIFT

(45) Veröffentlichungstag der Patentschrift: **14.03.90**

(21) Anmeldenummer: **86108517.3**

(22) Anmeldetag: **23.06.86**

(51) Int. Cl.⁵: **C 07 D 249/18,**
C 07 D 207/408,
C 07 C 69/67, C 07 C 69/73,
C 07 C 69/757, C 07 C 69/76,
C 07 K 17/00

(54) **Verbrückungsreagenz, Verfahren zu seiner Herstellung und seine Anwendung.**

(30) Priorität: **29.06.85 DE 3523365**

(43) Veröffentlichungstag der Anmeldung:
**07.01.87 Patentblatt 87/02**

(45) Bekanntmachung des Hinweises auf die Patenterteilung:
**14.03.90 Patentblatt 90/11**

(84) Benannte Vertragsstaaten:
**AT BE CH DE FR GB IT LI LU NL SE**

(56) Entgegenhaltungen:
**EP-A-0 022 208**
**EP-A-0 064 714**
**EP-A-0 125 567**
**GB-A-1 059 674**
**GB-A-2 005 258**
**US-A-3 836 433**

**CHEMICAL ABSTRACTS, Band 87, Nr. 23, 5. Dezember 1977, Columbus, Ohio, USA HORIKI, KUSUO "Amino acids and peptides. Part 3. Behavior of acylated 1-hydroxybenzotriazole" Seite 651, Spalte 1, Zusammenfassung-Nr. 184 929k**

(73) Patentinhaber: **BEHRINGWERKE Aktiengesellschaft**
**Postfach 1140**
**D-3550 Marburg 1 (DE)**

(72) Erfinder: **Stüber, Werner, Dr.**
**Cölber Weg 12**
**D-3551 Lahntal 3 (DE)**

(74) Vertreter: **Klein, Otto, Dr. et al**
**Hoechst AG Zentrale Patentabteilung Postfach 80 03 20**
**D-6230 Frankfurt am Main 80 (DE)**

(56) Entgegenhaltungen:
**CHEMICAL ABSTRACTS, Band 86, Nr. 25, 20. Juni 1977, Columbus, Ohio, USA MC CARTY, DANIEL G.; HEGARTY, ANTHONY F.; HATHAWAY, BRIAN J. " N-Hydroxy-compounds as acyl transfer agents. Part 1. Kinetics and mechanism of nucleophilic displacements on 1-hydroxybenzotriazole esters and crystal and molecular structure of 1-benzoyloxybenzotriazole" Seite 509, Spalte 2, Zusammenfassung-Nr. 188 693f**

**EP 0 207 415 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(56) Entgegenhaltungen:

CHEMICAL ABSTRACTS, Band 90, Nr. 23, 4. Juni 1979, Columbus, Ohio, USA ITOH, MASUMI; NOJIMA, HIROSHI; NOTANI; JIYOJI; HAGIWARA, DAIJIRO; TAKAI, KUNIAKI "Peptides. VII. Some sulfonates of strongly acidic N-hydroxy compounds as novel coupling reagents" Seite 701, Spalte 2, Zusammenfassung-Nr. 187 317g

CHEMICAL ABSTRACTS, Band 97, Nr. 25, 20. Dezember 1982, Columbus, Ohio, USA TOYO JOZO CO., LTD. "Human parathyroid hormone(1-38) fragment." Seite 909, Spalte 1, Zusammenfassung-Nr. 216 732r

CHEMICAL ABSTRACTS, Band 97, Nr. 25, 20. Dezember 1982, Columbus, Ohio, USA PAQUET, ALENKA "Introduction of 9-fluorenylmethyloxycarbonyl, and benzyloxycarbonyl amine protecting groups into O-unprotected hydroxy amino acids using succinimidyl carbonates" Seite 900, Spalte 1, Zusammenfassung-Nr. 216 635m

BEILSTEINS HANDBUCH DER ORGANISCHEN CHEMIE, 4. Auflage, Zweites Ergänzungswerk, Band 3/4, 1942 SPRINGER-VERLAG, Berlin Seite 411

BEILSTEINS HANDBUCH DER ORGANISCHEN CHEMIE, 4. Auflage, Zweites Ergänzungswerk, Band3/4, 1942 SPRINGER-VERLAG, Berlin Seite 434

PATENT ABSTRACTS OF JAPAN, unexamined applications, Field C, Band 7, Nr. 143, 22. Juni 1983 THE PATENT OFFICE JAPANESE GOVERNMENT Seite 172
Methoden der Organischen Chemie (Houben-Weyl), Band VIII, Sauerstoffverbindungen III, Georg Thieme Verlag 1952, Seite658-660

**Beschreibung**

Der Bedarf an Verbrückungsreagenzien zur Kupplung von Aminosäuren, Peptiden oder biologischen Wirkstoffen an Proteine und inerte Träger nimmt ständig zu. Hierzu sind heterobifunktionelle Reagenzien mit der allgemeinen Formel B entwickelt worden, mit welchen z.B. Peptide spezifisch N-terminal an Proteine und andere Träger gekuppelt werden können (Christner, J.: Synthese von Peptiden zur immunologischen Bestimmung von Proteinen; Dissertation 1984, Universität Tübingen).

V—NH—NH—CO—A—CO—Y *B*

Es bedeutet:
V = Boc, *Z, Fmoc* oder andere Schutzgruppen
Y = OH, OSu oder andere Aktivestergruppen
A = Ethylen- oder Propylengruppe.

Oben und im folgenden Text genannte Abkürzungen sind in einer Liste zusammengefaßt.

Kupplungen mit solchen Reagenzien können in einer mehrstufigen Arbeitsweise ausgeführt werden. Die aktivierte Carboxygruppe reagiert in erster Stufe mit Aminogruppen einer bestimmten Verbindung, z.B. einem Polymer oder einem Peptid. In den darauffolgenden Schritten wird die Schutzgruppe V abgespalten, das entstandene Hydrazid ins Azid überführt, welches man dann unter schonenden Bedingungen mit der Aminogruppe einer anderen Komponente reagieren läßt. Dieses Verfahren ist jedoch umständlich und zeitaufwendig. Es ergibt sich daher der Bedarf an Aktivierungsreagenzien, die in der Lage sind, Moleküle mit Aminogruppen selektiv und einfach an ein Trägermolekül zu binden.

Dieses Problem konnte mit der vorliegenden Erfindung gelöst werden, indem man Aldehydsäuren, deren Carboxygruppe in einen geeigneten kupplungsfähigen Zustand gebracht wurde, während die Carbonylgruppe ungeschützt vorliegt, als Verbrückungsreagenzien einsetzt und in einem zweistufigen Verfahren selektiv mit Aminogruppen-tragenden Substanzen zur Reaktion bringt.

Die Erfindung betrifft somit:

1. Eine Verbindung der allgemeinen Formel I,

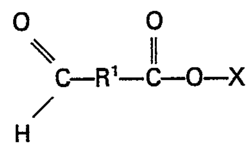

I

in der
$R^1$ ein aliphatischer oder aromatischer Rest mit bis zu 10 C-Atomen und
X ein Succinimid- oder Benzotriazolrest ist.

2. Ein Verfahren zur Herstellung der Verbindung der allgemeinen Formel I, die erhalten wird, indem eine Verbindung der allgemeinen Formel II,

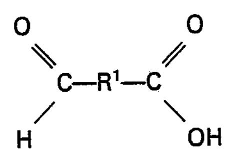

II

in der $R^1$ die obengenannte Bedeutung hat, mit einer Verbindung der allgemeinen Formel III,

XOH III

in X die obengenannte Bedeutung hat, zusammen mit einem Kondensationsmittel unter Abspaltung von Wasser umgesetzt wird.

3. Die Verwendung der Verbindung der allgemeinen Formel I
a) als bifunktionelles Kupplungsreagenz,
b) zur Herstellung von Amiden mit Aldehydfunktion
c) Zur Herstellung von Schiffschen Basen.

In der folgenden Beschreibung und in den Patentansprüchen werden bevorzugte Ausgestaltungen der Erfindung aufgeführt.

Als Aldehydsäuren (Verbindung der Formel II) können aliphatische und aromatische Verbindungen mit bis zu 10 C-Atomen, 10 H-Atomen und 5 O-Atomen eingesetzt werden, wobei aromatische Aldehydsäuren, insbesondere 4-Carboxybenzaldehyd, bevorzugt sind. Zur Aktivierung der Carboxygruppen werden die in der Peptidchemie üblichen Methoden benutzt. Bevorzugt geschieht die Aktivierung durch Bildung von symmetrischen Anhydriden oder von sogenannten Aktivestern mit aktivesterbildenden Molekülen, wie sie beispielsweise in M. Bodanszky, Principles of Peptide Synthesis, Springer Verlag, 1984, beschrieben werden, insbesondere mit N-Hydroxysuccinimid oder Hydroxybenzotriazol. Die Reaktion wird in Gegenwart von Carbodiimiden, vorzugsweise Dicyclohexylcarbodiimid, durchgeführt.

Diese aktivierten Verbindungen werden dann zusammen mit einer Aminogruppen-tragenden Substanz

in einem polaren organischen Lösemittel suspendiert oder gelöst. Hier sind beispielsweise Dioxan, Tetrahydrofuran, Ethanol, Dimethylsulfoxid, Methylenchlorid, Trichlormethan oder ganz besonders Dimethylformamid geeignet. Dem Reaktionsgemisch wird eine Base hinzugegeben, wobei sich tertiäre Amine, insbesondere N-Methylmorpholin oder Triethylamin, besonders bewährt haben.

Die Reaktion wird so ausgeführt, daß die Aminogruppentragende Substanz äquimolar oder im Unterschuß vorliegt. Gute Umsatzraten lassen sich erhalten, wenn das Molverhältnis der Aminokomponente zum Kupplungsreagenz in dem Bereich 1:1 bis 1:10, bevorzugt bei 1:1 bis 1:3, gehalten wird.

Überraschenderweise kuppelt die Aminogruppe bei dieser Reaktion selektiv mit der aktivierten Carboxygruppe der erfindungsgemäßen Verbindung und nicht mit der Carbonylgruppe. Es lassen sich somit Amide mit Aldehydfunktion gewinnen, die in einem weiteren Reaktionsschritt selektiv an andere Aminogruppen-tragende Verbindungen gekuppelt werden können. Zu diesem Zweck können die Amide isoliert werden. Bei Peptiden geschieht dies bevorzugt durch Extraktion oder Fällung und bei polymeren Trägern durch Waschen mit Lösemitteln.

Zur Anknüpfung an eine zweite Aminokomponente werden die Reaktionspartner in einer wäßrigen Lösung zusammengegeben, die gegebenenfalls in einem pH-Bereich von 5 bis 10, bevorzugt 6 bis 9, gepuffert ist, und über einen Zeitraum von 2 Stunden bis 7 Tage, bevorzugt 1 Tag, inkubiert. Die dabei auftretenden Schiffschen Basen sind im Falle aromatischer Aldehyde mit einer genügenden Stabilität gegen Hydrolyse ausgestattet. Insbesondere bei weniger hydrolysestabilen Schiffschen Basen kann eine Hydrierung der Aldimingruppe zur sekundären Aminogruppe erfolgen. Die Reduktion mit komplexen Hydriden, z.B. mit Natriumcyanoborhydrid, erwies sich als günstig bezüglich der Verkürzung der Reaktionszeit.

Die Vorteile des erfindungsgemäßen Verfahrens liegen in seiner Schnelligkeit, in der selektiven Umsetzung bei den Kupplungsreaktionen, den hohen Ausbeuten und Stabilitäten der Produkte. Ein weiterer Vorteil liegt darin, daß die Amide mit Aldehydfunktion unter entsprechenden Bedingungen, gegebenenfalls unter Einführen einer Spacergruppe, über Kupplung an den N-Terminus mit weiteren Aminosäuren umgesetzt werden können und so Peptide aufgebaut werden können.

Weitere detaillierte Ausgestaltungen werden im folgenden anhand von Beispielen dargestellt.

### Beispiel 1

Immobilisierung von H-ε-Aca-Gly-Ala-Lys(Boc)-Gln-Ala-OMe an aminiertes Harz

*Stufe 1*

150 mg 4-Carboxybenzaldehyd und 153 mg 1-Hydroxybenzotriazol-(1,2,3) werden in 5 ml DMF gelöst und mit 210 mg Dicyclohexylcarbodiimid versetzt. Es wird 1 Stunde bei Raumtemperatur gerührt, bevor 110 µl N-Methylmorpholin und 736,5 mg H-ε-Aca-Gly-Ala-Lys(Boc)-Gln-Ala-OMe (in DMF vorgelöst) zugesetzt werden. Die Reaktionsmischung wird über Nacht bei Raumtemperatur gerührt. Das Lösemittel wird abgedampft, der Rückstand mit Essigester und Dichlormethan ausgerührt und abfiltriert. Der Rückstand wird gesammelt und getrocknet.

Ausbeute: 720 mg ≙ 83% d. Th.

Reinheitskontrollen: DC $R_f = 0,37$

Laufmittel: Chloroform/Methanol/Essigsäure (5:2:0,5; v:v:v)

Detektion: Ninhydrin, UV, TDM und 2,4-Dinitrophenylhydrazin positiv

*Stufe 2*

10 ml einer Suspension eines Copolymerisats mit Aminogruppen aus Pentaerythrit, Methacrylsäurederivaten und Polyethylenglykol, vernetzt mit Divinylbenzol, (Fractogel® TSK HW 65 (F) ≙ 1,5 g trockene Substanz mit 0,18 mmol Aminogruppen in wäßriger Suspension) wird mit 1 M Natriumphosphatpuffer auf pH 9 gebracht und mit 347 mg des nach Stufe 1 aktivierten Peptids versetzt. Der Ansatz wird 24 Stunden bei Raumtemperatur geschüttelt. Anschließend wird das Harz mit Wasser und Methanol gewaschen, Eine Peptidgehaltsanalyse erbrachte 0,033 mmol Peptid pro g trockenem Harz.

### Beispiel 2

Herstellung von aminiertem Harz mit Aldehydfunktion

10 ml der in Beispiel 1 genannten Polymer-Suspension (≙ 1,5 g Trockensubstanz) wird mit Dioxan-Wassergemischen steigender Dioxankonzentration entwässert. Separat werden 150 mg 4-Carboxybenzaldehyd und 153 mg 1-Hydroxybenzotriazol(-1,2,3) in 5 ml DMF gelöst und mit 210 mg Dicyclohexylcarbodiimid versetzt. Der Ansatz wird 1 Stunde bei Raumtemperatur gerührt und dann über eine G 3-Glasfilterfritte zu dem in 10 ml Dioxan suspendierten Harz gegeben. Man fügt 100 µl N-Methylmorpholin hinzu und schüttelt den Ansatz über Nacht. Überschüssige Reagenzien werden durch Filtration und Waschen mit Dioxan entfernt. Das Harz wird mit Wasser-Dioxan-Mischungen steigender Wasserkonzentration in eine rein wäßrige Phase überführt.

### Beispiel 3

Anlagerung von biologisch aktiven Substanzen an aminiertes Harz mit Aldehydfunktion

0,5 g des gemäß Beispiel 2 hergestellten Harzes mit ca. 0,05 mmol Aldehydgruppen pro Gramm Polymer werden in 3 ml 1 M Natriumphosphatpuffer pH 9 suspendiert und mit 100 mg H-Arg(HCl)-ANBS-

Lys(Z)-OMe versetzt. Man schüttelt 2 Tage bei Raumtemperatur und wäscht überschüssige Reagentien mit Wasser aus.

Beladungsprüfung: Nach Trocknung des Harzes ergab eine Aminosäurenanalyse 0,05 mmol/g Beladung.

Beispiel 4

Konjugation von H-ε-Aca-Glu-Glu-Asn-Gly-Leu-Ala-Leu-OH an KLH

*Stufe 1*

7,5 mg 4-Carboxybenzaldehyd und 7,65 mg 1-Hydroxybenzotriazol-(1,2,3) werden in 2 ml Dimethylformamid gelöst und mit 10,5 mg Dicyclohexylcarbodiimid versetzt. Der Ansatz wird eine Stunde bei Raumtemperatur gerührt und mit 20 µl N-Methylmorpholin versetzt. Darauf hin werden 56 mg H-ε-Aca-Glu-Glu-Asn-Gly-Leu-Ala-Leu-OH × 3 $H_2O$ × ACOH, die in 2 ml Dimethylformamid gelöst waren, langsam zugegeben. Die Reaktionsmischung wird über Nacht bei Raumtemperatur gerührt, das Lösemittel abgedampft und der Rückstand zwischen Diethylether und Wasser verteilt. Nach Lyophilisation der Wasserphase erhält man das Peptid mit Aldehydfunktion in quantitativer Ausbeute in Form eines lockeren, schneeweißen Pulvers.

Reinheitsprüfung: DC: $R_f = 0,53$
Laufmittel: Butanol/Essigsäure/Wasser (3:1:1; v:v:v)
Detektion: Ninhydrin negativ
UV, 2,4-Dinitrophenylhydrazintest und TDM positiv

zum Vergleich, DC freies Peptid: $R_F = 0,12$
Laufmittel: s.o.
Detektion: TDM und Ninhydrin positiv
UV und 2,4-Dinitrophenylhydrazin negativ

*Stufe 2*

25 mg des nach Stufe 1 aktivierten Peptids werden in 4 ml Phosphatpuffer (0,5 M, pH 8,3) gelöst und mit 125 mg KLH versetzt. Hierzu gibt man 1,5 mg festes Natriumcyanoborhydrid und rührt den Ansatz über Nacht. Das Konjugat wird durch Dialysieren gegen destilliertes Wasser gereinigt und anschließend durch Lyophilisation in fester Form gewonnen.

*Abkürzungen*

| | |
|---|---|
| Lys | L-Lysin |
| ε-Aca | ε-Aminocapronsäure |
| Gly | Glycin |
| Ala | L-Alanin |
| Gln | L-Glutamin |
| Ome | Methylester |
| DC | Dünnschichtchromatographie |
| DMF | Dimethylformamid |
| $R_F$ | Retentionsfaktor |
| UV | Ultraviolettvisualisation |
| TDM | (Chlor/4,4-Bis-(dimethylamino)diphenylmethan-Test |
| Arg | L-Arginin |
| ANBS | 5-Amino-2-nitrobenzoesäure |
| Z | Benzyloxycarbonyl- |
| Glu | L-Glutaminsäure |
| Leu | L-Leucin |
| Asn | L-Asparagin |
| KLH | Keyhole Limpet Hemocyanine |
| ACOH | Essigsäure |
| Boc | tert.-Butyloxycarbonyl |
| Fmoc | 9-Fluorenylmethyloxycarbonyl |
| HOSu | N-Hydroxysuccinimid |

**Patentansprüche**

1. Verbindung der allgemeinen Formel I,

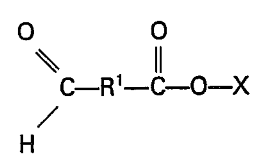

I

in der

$R^1$ ein aliphatischer oder aromatischer Rest mit bis zu 10 C-Atomen und

X ein Succinimid- oder Benzotriazolrest ist.

2. Verbindung nach Anspruch 1, dadurch gekennzeichnet, daß $R^1$ der p-Phenylenrest ist.

3. Verfahren zur Herstellung einer Verbindung nach mindestens einem der Ansprüche 1 und 2, dadurch gekennzeichnet, daß eine Verbindung der allgemeinen Formel II,

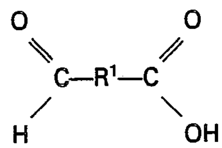

II

in der $R^1$ die obengenannte Bedeutung hat, mit einer Verbindung der allgemeinen Formel III,

$$XOH \qquad III$$

in der X die obengenannte Bedeutung hat, zusammen mit einem Kondensationsmittel unter Abspaltung von Wasser umgesetzt wird.

4. Verfahren nach Anspruch 3, dadurch gekennzeichnet, daß das Kondensationsmittel ein Carbodiimid ist.

5. Verfahren nach Anspruch 4, dadurch gekennzeichnet, daß das Kondensationsmittel Dicyclohexylcarbodiimid ist.

6. Verwendung einer Verbindung nach mindestens einem der Ansprüche 1 und 2 zur Kopplung zweier Aminogruppen-tragender Verbindungen.

7. Verwendung einer Verbindung nach mindestens einem der Ansprüche 1 und 2 zur Herstellung von Amiden mit Aldehydfunktion.

8. Verwendung einer Verbindung nach mindestens einem der Ansprüche 1 und 2 zur Herstellung von Schiffschen Basen.

**Revendications**

1. Composé de formule générale I

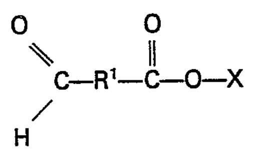

I

dans laquelle

$R^1$ est un radical aliphatique ou aromatique ayant jusqu'à 10 atomes de carbone et

X est un reste succinimide ou benzotriazole.

2. Composé selon la revendication 1, caractérisé en ce que $R^1$ est le radical p-phénylène.

3. Procédé pour la préparation d'un composé selon au moins l'une des revendications 1 et 2, caractérisé en ce que l'on fait réagir un composé de formule générale II

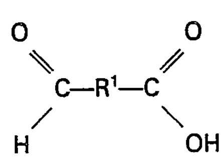

II

dans laquelle $R^1$ a la signification donnée plus haut, avec un composé de formule générale III

$$XOH \qquad III$$

dans laquelle X a la signification donnée plus haut, conjointement avec un agent de condensation, avec élimination d'eau.

4. Procédé selon la revendication 3, caractérisé en ce que l'agent de condensation est le carbodiimide.

5. Procédé selon la revendication 4, caractérisé en ce que l'agent de condensation est le dicyclohexylcarbodiimide.

6. Utilisation d'un composé selon au moins l'une des revendications 1 et 2, pour le couplage de deux composés portant des groupes amino.

7. Utilisation d'un composé selon au moins l'une des revendications 1 et 2, pour la préparation d'amides à fonction aldéhyde.

8. Utilisation d'un composé selon au moins l'une des revendications 1 et 2, pour la préparations de bases de Schiff.

## Claims

1. A compound of the general formula I,

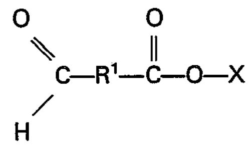

I

in which
$R^1$ is an aliphatic or aromatic radical having up to 10 carbon atoms and
X is a succinimide or benzotriazole residue.

2. A compound as claimed in claim 1, wherein $R^1$ is the p-phenylene radical.

3. A process for the preparation of a compound as claimed in at least one of claims 1 and 2, which comprises reacting a compound of the general formula II,

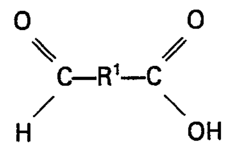

II

in which $R^1$ has the abovementioned meaning, with a compound of the general formula III,

XOH III

in which X has the abovementioned meaning, together with a condensing agent, with elimination of water.

4. The process as claimed in claim 3, wherein the condensing agent is a carbodiimide.

5. The process as claimed in claim 4, wherein the condensing agent is dicyclohexylcarbodiimide.

6. The use of a compound as claimed in at least one of claims 1 and 2 for coupling two compounds carrying amino groups.

7. The use of a compound as claimed in at least one of claims 1 and 2 for the preparation of amides having an aldehyde function.

8. The use of a compound as claimed in at least one of claims 1 and 2 for the preparation of Schiff's bases.